(19) Europäisches Patentamt
European Patent Office
Office européen des brevets

(11) **EP 4 257 037 B1**

(12) **EUROPEAN PATENT SPECIFICATION**

(45) Date of publication and mention
of the grant of the patent:
**18.03.2026   Bulletin 2026/12**

(21) Application number: **22166766.0**

(22) Date of filing: **05.04.2022**

(51) International Patent Classification (IPC):
*A61B 5/0537* (2021.01)     *A61B 5/0538* (2021.01)
*A61B 5/145* (2006.01)      *A61B 5/00* (2006.01)

(52) Cooperative Patent Classification (CPC):
**A61B 5/0538; A61B 5/0537; A61B 5/14503;**
**A61B 5/14532;** A61B 5/0031; A61B 2560/0219;
A61B 2560/0406; A61B 2560/0468; A61B 2562/043;
A61B 2562/18

(54) **IMPLANTABLE SENSOR WITH OPTIMAL ELECTRODE DISTANCE**

IMPLANTIERBARER SENSOR MIT OPTIMALEM ELEKTRODENABSTAND

CAPTEUR IMPLANTABLE À DISTANCE D'ÉLECTRODE OPTIMALE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**11.10.2023   Bulletin 2023/41**

(73) Proprietor: **D.T.R. Dermal Therapy Research Inc.**
**London, ON N5X 2N9 (CA)**

(72) Inventors:
• **OLLMAR, Stig**
**141 46 HUDDINGE (SE)**

• **DUENAS, Saul Alejandro Rodriguez**
**176 68 JÄRFÄLLA (SE)**
• **BIRGERSSON, Ulrik**
**117 65 STOCKHOLM (SE)**

(74) Representative: **Groth & Co. KB**
**P.O. Box 6107**
**102 32 Stockholm (SE)**

(56) References cited:
**US-A- 5 970 986**      **US-A1- 2012 053 441**
**US-A1- 2015 196 224**      **US-A1- 2017 128 053**

EP 4 257 037 B1

## Description

### *Technical Field*

The invention relates to the field of implantable sensors for electrical measurements in the body. In particular the invention relates to implantable sensors for the determination of glucose concentration that apply four-point measurement for the determination of impedance in the body tissue, which can be muscle tissue.

### *Background of the Invention*

Known sensors for implanting in the body typically contain a printed circuit board, a power source and electrodes or chemical electrodes for determining various parameters in the body. Many known sensors have the aim to detect rejection of an organ after organ transplantation by measuring the trend of the impedance in the transplanted organ after transplantation. A sign of organ rejection is an increase (trend) in electric impedance in an organ (organ tissue) after transplantation. Once such an increase started, a doctor can apply drugs to a certain extent, but if a threshold is reached then the organ will be rejected and basically die within the recipient body. Such a sensor is for instance illustrated in the US 5,970,986 B1.

In the US 5,970,986 B1 an apparatus for rejection diagnostics after organ transplantations, which is equipped with an extra corporal base station comprising a high frequency transmitter and receiver unit that can transmit data to an implantable rejection sensor, is disclosed. The implantable rejection sensor comprises an integrated circuit component, thus an IC component, to which a receiving- and transmitting coil are allocated. The implantable rejection sensor further comprises a sensor arrangement comprising of four electrodes arranged on the plane surface of the housing. US 5,970,986 B1 further states that the electrodes and the coils can be integrated in the IC component. The implantable rejection sensor is configured to be fixed onto a donor organ.

It is herewith important to note that the sensor disclosed in US 5,970,986 B1 is for measuring organ rejection and when doing so the parameter that matters is the trend of the impedance in the organ as explained above. The absolute values of impedance are not critical when detecting organ rejection.

The present disclosure however relates to the determination of glucose levels or glucose concentration in body fluids or tissue of a human or animal and thus a living being by correlating measured impedance values in the body fluid or tissue to a database containing a matrix having values of impedance values correlated to glucose levels or glucose concentration.

When glucose concentration is determined in the tissue/body of a living being, which can be summarized with the term living beings, the absolute impedance values are of interest since the absolute impedance values correlate to glucose concentration. If these measured values are false then the correlated glucose values are false, which can lead to unwanted consequences when administering insulin.

One issue that falsifies the measured impedance once a sensor is implanted in the body of a living being, is body liquid that collects around certain areas around the implanted sensor and around the electrodes. This body liquid can be blood, wound liquid or other extra cellular liquids or a mixture thereof. Since the electric conductivity is better in liquids, these body liquids affect and falsify the measured impedance and therefore affect the accuracy of the measurement. Tests and reference measurements have shown that body liquids anywhere around the implanted sensor affect the accuracy of the impedance measurements substantially.

The sensor shown in the US 5,970,986 B1 has exactly this problem due its sharp edges and flat surfaces. However, since US 5,970,986 B1 is concerned with detecting organ rejection, this problem is not mentioned in the document.

Another problem of many known sensors is that the coil for receiving the magnetic field from a power source for powering the sensor is arranged on, under or above the printed circuit board (PCB) of the sensor. Since the PCB typically contains conducting loops and the like, the magnetic field induces Eddy Currents in the PCB, which Eddy Currents can affect the measurement performance and disturb the energy or power transfer from the power source to the sensor by reducing the allowable or workable distance between the implanted sensor and the external reader and power source, respectively.

Still another issue of known prior art solutions is that the measurement of the impedance needs to be performed in healthy tissue. Typically, when sensors are implanted in the body of a living being, scar tissue will develop around the sensor once all the wounds are healed. This scar tissue is however not representative of the impedance in healthy tissue, since there is less blood flow in the scar tissue. Likewise other body liquids will only enter the scar tissue via diffusion. Therewith it is of importance to pass this scar tissue when measuring the impedance with an implanted sensor. In other words, the impedance should be measured in healthy tissue, since impedance values measured in the scar tissue are not representative of body fluid parameters.

An implantable impedance sensor for measuring glucose levels is for example known from document US2015/196224A1.

### *Summary of the Invention*

In view of the above it is an object of the present invention to provide an improved sensor for determining glucose concentration in a living being, so that the impedance measurement within tissue of a living being where the sensor is implanted is exact and can be correlated to a database containing glucose concentrations correlated to reference values of impedance.

It is another object of the invention to provide a sensor that is reliable and safe.

It is a further object of the invention to provide a sensor that is easy to handle for a patient.

In view of the above-mentioned problems and objects the inventors of the present invention have discovered that the thickness of scar tissue in the body of a living being, for example a human, is normally about 2mm and that it develops at least more or less homogenously around the implanted sensor once the wounds from the implantation of the sensor have healed. Depending on this thickness of the scar tissue, the inventors discovered that the spacing of the current injecting electrodes has to be in a range of 5mm to 12mm, preferably about 6 to 10 mm and more preferably about 7 to 9mm, whilst the voltage sensing electrodes are placed in between the injecting electrodes and at least more or less on a straight line. The voltage sensing electrodes may also be positioned outside the straight line between the current injection electrodes, e.g. in the corners of a square or rectangle.

In a second aspect the inventors of the present invention have realized that it is possible to arrange the coil that is used to power the implanted sensor via an external device using a pulsed or varying magnetic field, outside of the outer housing of the implantable sensor and connect the coil via a conductor to the electronics, thus the circuit board, of the sensor. This means that the coil can be positioned close to the skin of the patient for easy powering during glucose level determination, while the sensor can be placed a bit deeper in the body of the living being. This can improve measurement results, as explained later herein, and protect the sensor better at the same time.

The above-mentioned distance between the current injecting electrodes has the effect that the current trajectories that occur during current injection by the current injection electrodes extend beyond the scar tissue. As long as the distance is more than 4mm the current trajectories will extend beyond the scar tissue. However, there are also limitations to the maximal distance, since the greater that distance is the more will body movements of the living being and inhomogeneous tissue affect measurement results. This means that the optimal distance is rather limited and it has been shown by experiments and testing that the above described and claimed range herein improves measurement results substantially.

The inventors of the present invention have further realized that it is necessary to space the voltage sensing electrodes about 1mm to 4mm, preferably about 2-3mm from the current injecting electrodes for optimal measuring results. The current injecting electrodes and the voltage sensing electrodes may be positioned, at least more or less, on a straight line, or sideways from a straight line, displaced versus or in line with the straight line.

This spacing relating to the measurement electrodes is optimal in order to make sure that the current injection from the current injecting electrodes is not affecting the measurement and that the impedance can be measured over a reasonable distance so that a feasible value can be returned. The closer the voltage sensing electrodes are positioned to one another, the less voltage signal would be detected, which would reduce the signal to noise ratio, something that is undesirable. The signal to noise ratio should always be high to have a high-quality signal.

According to a first aspect, disclosed herein is an implantable glucose sensor for determining impedance in tissue of a living being and correlating the measured impedance with known glucose concentrations and impedance values of a database, the implantable glucose sensor comprises:

- a housing,
- two current injecting electrodes for injecting current into the tissue,
- two voltage sensing electrodes for measuring impedance in the tissue, whereby the two voltage sensing electrodes are arranged separately from the two current injecting electrodes,
- a coil for powering the implantable glucose sensor via a power source,
- a circuit board arranged within the housing, the circuit board being electrically connected to the two voltage sensing electrodes and the two current injecting electrodes and the coil,
- a communication unit for transferring and receiving data packages, the communication unit being connected to the circuit board and whereby,
- the housing comprises the two voltage sensing electrodes and the two current injecting electrodes on an outer surface.

In the implantable sensor a distance (d) between the two current injecting electrodes on the outer surface is in a range from 5mm to 12mm, preferably about 6 to 10 mm and more preferably about 7 to 9mm.

The above specified sensor and current injecting electrode distancing improves the measurement results and ensures that the impedance in healthy undamaged tissue is measured and only minimally diluted or affected by the impedance of scar tissue around the implanted sensor. The scar tissue is not as well nourished with body liquids and blood instead it is only nourished by diffusion and therewith the impedance values from scar tissue are not representative as accurate values. The healthy tissue however is representative of impedance values since body liquids and blood is flowing through this tissue unhindered.

In an embodiment the relationship between the distance (d) between the two current injecting electrodes and a distance (e) between one of the two voltage sensing electrodes and a closest current injecting electrode and a distance between the other of the two voltage sensing electrodes and a closest current injecting electrode, respectively follows the equation:

$$e < d/2 \text{ and } e \geq 1\text{mm}.$$

Following the above equation will provide a good outcome for the impedance measurement, as explained later herein.

In an embodiment the voltage sensing electrodes may be arranged symmetrically in between the injecting electrodes and wherein a distance (d) between one of the two voltage sensing electrodes and a closest current injecting electrode and a distance (d') between the other of the two voltage sensing electrodes and a closest current injecting electrode **4'** is in a range from 1mm to 4mm, preferably about 2mm to 3mm.

Such a distancing between the two current injecting electrodes and the two voltage sensing electrodes improves the measurement since the voltage sensing electrodes do not pick up ill-defined values from the immediate neighborhood of the injecting electrodes due to distorted injected current density and since the distance between the two voltage sensing electrodes is still reasonable for measuring impedance.

In an embodiment the two current injecting electrodes further comprise a first active area made of conductive material and wherein the voltage sensing electrodes comprise a second active area made of conductive material.

The size of the first active area ensures that enough amperage or voltage can be transferred into the tissue so that a high-quality measurement can be achieved, while keeping the current density in the tissue low enough to avoid non-linear effects.

In another embodiment the first active area and the second active area may be made of gold, or other biocompatible conductive material.

In still another embodiment the two current injecting electrodes may have a longitudinal shape, such as for example an elliptic longitudinal shape or a rectangular longitudinal shape.

In another embodiment the housing may be shaped harmonic and rounded off and convex on a bottom side and on a top side.

The housing may be of an ellipsoid shape.

This may reduce any accumulation of liquids around the implantable sensor.

In still another embodiment the implantable sensor may have outer dimensions of the implantable glucose sensor corresponding to a length of 10mm to 50mm, preferably 20mm to 30mm, a width of 5mm to 25mm, preferably 11mm to 15mm and a thickness of 1mm to 15mm, preferably 2mm to 5mm.

The implantable sensor is an implantable glucose sensor.

According to a second aspect, disclosed herein is further an implantable sensor for determining impedance in tissue of a living being, the implantable sensor comprising:

- a housing,
- two current injecting electrodes for injecting current into the tissue,
- two voltage sensing electrodes for measuring impedance in the tissue, whereby the two voltage sensing electrodes are arranged separately from the two current injecting electrodes,
- a coil for powering the implantable glucose sensor via a power source,
- a circuit board arranged within the housing, the circuit board being electrically connected to the two voltage sensing electrodes and the two current injecting electrodes and the coil,
- a communication unit for transferring and receiving data packages, the communication unit being connected to the circuit board, and whereby,
- the housing comprises the two voltage sensing electrodes and the two current injecting electrodes on an outer surface.

The coil may be connected to the circuit board, the two voltage sensing electrodes, the two current injecting electrodes and the circuit board by conductors.

In an embodiment the coil may be arranged separately from the housing or outside the housing.

In still another embodiment the connector that connects the coil with the circuit board, the two voltage sensing electrodes and the two current injecting electrodes may be a flexible cable.

In still another embodiment the implantable sensor may comprise elements for fastening the coil to tissue of the living being.

The above-described embodiment improves the energy transfer from an external device and a coil of the external device, respectively, to the coil of the implantable sensor. This means that the coil of the sensor can be positioned and for instance sewn to the tissue right under the skin (or fur) or at least close to the skin so that the coil of the implantable sensor is at least more or less parallel to an outer surface of the skin. Since the coil is rather small, in a range of about 6 mm to 15mm in diameter, preferably 7 mm to 12mm in diameter and more preferably about 8 mm to 10mm it is rather simple to sew the coil close to the skin.

Having the possibility to place the implanted sensor further away from its coil solves two issues, namely:

i) that during the powering of the coil via the external device, Eddy Currents can basically not affect the other electronic components of the sensor, such as the circuit board, the powering and communication unit, the two voltage sensing and the two current injecting electrodes, and

ii) no energy is lost by induced Eddy Currents produced by the pulsed or varying magnetic field through the coil, and

iii) that the alignment between the coil of the external device and the coil of the implantable sensor is simplified.

According to a further embodiment according to the disclosure herein, the implantable sensor may be an implantable glucose sensor and wherein a glucose concertation in the tissue is determined by correlating a measured impedance to a database containing impedance values and corresponding glucose concentrations.

For an improved alignment according to item iii) above it might further be conceivable to mark the position of the coil of the implantable sensor under the skin with a tattoo or a sticker on the skin so that the patient knows exactly where to put the external device when doing a measurement.

The above-described solutions and embodiments referring to the distancing and positioning of the current injecting electrodes and the voltage sensing electrodes may also be combined in one single solution of an implantable sensor. Thus, the external coil of the implantable sensor may be used with the specified electrode spacing or alternatively it may be used separately.

Explanation of Terms

Below certain expressions and terms used herein are explained and defined.

Distance

A distance in the context herein as measured on the housing of the sensor is to be interpreted as a distance between two items, objects or points measured on an outer surface of the housing, even if such a housing is convex or even slightly convex.

Range

The term range herein means that the starting number and the ending number given in the range are included in the range. Further the term mm herein refers to Millimeter.

Bio-compatible conductive material

The term bio-compatible conductive material herein refers to any bio-compatible conductive material or alloy, such as Gold, Titan, Platinum and so on or any alloy thereof.

Symmetric or Symmetrically

The term symmetric or symmetrically or symmetry herein refers to any symmetry along a center point or center line. It may thus include bilateral symmetry or 3-dimensional symmetry versus a plane that extends through a center of an arrangement, in the present case of the two current injecting electrodes and the two voltage sensing electrodes. The voltage sensing electrodes and the current injecting electrodes may further be arranged along a straight line on the housing of the sensor or they may at least be arranged in one single plane, or e.g. in the corners of a square or rectangle.

Generally, all terms used in the claims are to be interpreted according to their ordinary meaning in the technical field, unless explicitly defined otherwise herein. All references to "a/an/the element, apparatus, part, half, component, means, device, sensor and so on are to be interpreted openly as referring to at least one instance of the element, apparatus, part, half, component, means, device, sensor and so on unless explicitly stated otherwise. The steps of any method disclosed herein do not have to be performed in the exact order disclosed, unless explicitly stated.

***Brief Description of the Drawings***

The present invention will now be described, for exemplary purposes, in more detail by way of an embodiment(s) and with reference to the enclosed drawings, in which:

Fig. 1a      schematically illustrates a perspective view of an ellipsoidal implantable glucose sensor;

Fig. 1b      schematically illustrates a perspective view similar as figure 1, illustrating a distance (d) between the current injecting electrodes well illustrated;

Fig. 2a      schematically illustrates a perspective view of an embodiment similar as the one shown in figure 1a;

Fig. 2b      schematically illustrates a perspective view of an embodiment similar as the one shown in figure 2a, illustrating a distance (d') between the current injecting electrodes;

Fig. 3      schematically illustrates a graphic representation of current trajectories when current is injected in organic tissue via a pair of current injecting electrodes;

Fig. 4      schematically illustrates the interior of an implantable sensor with certain parts omitted for illustrative purposes;

Fig. 5      schematically illustrates the powering of the implantable sensor of figure 2a during measuring of the impedance; and

Fig. 6      schematically illustrates another embodiment of the sensor according to the invention having a coil arranged outside a housing of the sensor.

*Detailed Description*

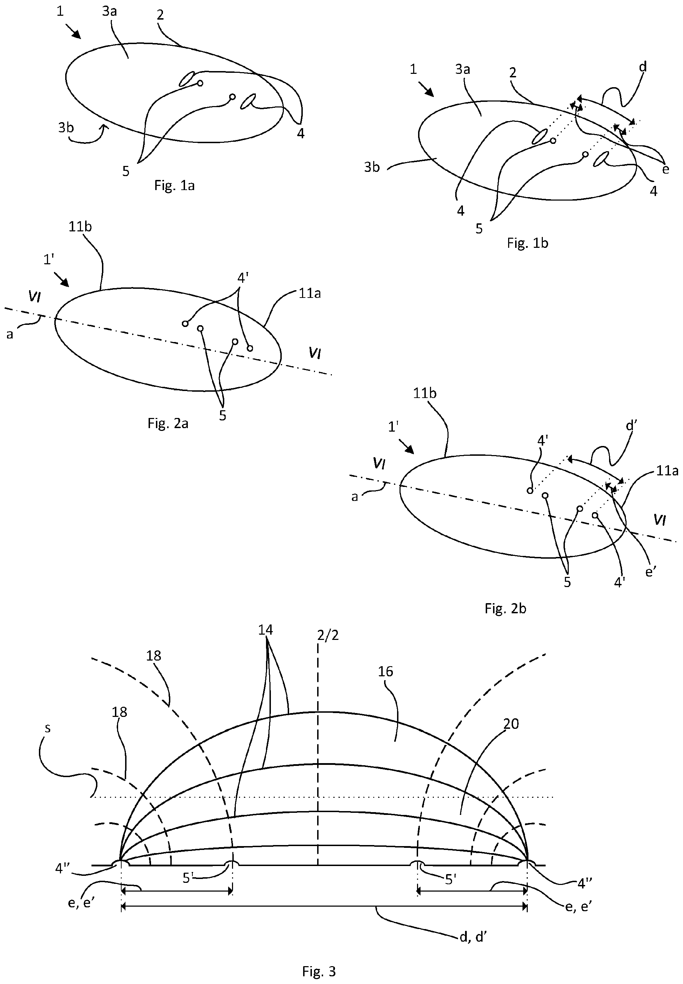

Figure 1a illustrates an implantable sensor 1 comprising a housing 2 of double convex, ellipsoidal or ellipsoidal shape or just in general a rounded off and harmonic shape. The housing comprising a first part 3a and a second part 3b. The first part 3a, which could be considered the upper first part 3a, comprises two current injecting electrodes 4 and two voltage sensing electrodes 5. The current injecting electrodes 4 are configured to inject current and the voltage sensing electrodes 5 are configured to measure voltage and so determine impedance. The second part 3b, which could be considered the lower second part 3b, comprises fastening means (not shown) for fastening the implantable sensor 1 to tissue. The fastening means can be employed in the form of loops or hooks. The current injecting electrodes 4 and voltage sensing electrodes 5 are integrated in the housing 1 and arranged smooth and flush with an outer surface of the first part 3a.

The current injecting electrodes 4 are of a longitudinal shape or ellipsoid shape, whereas the voltage sensing electrodes 5 are shown circular.

From figure 1a it can be seen that the voltage sensing electrodes 5 are arranged rather close to the current injection or current injecting electrodes 4. With reference to figure 1b the optimal distances between the pair of current injection electrodes 4 and the optimal distance between the voltage sensing electrodes 5 and the current injecting electrodes 4 will be further explained and discussed.

Figure 1b shows the same or at least a very similar embodiment as figure 1a with a distance (d) indicated. The distance (d) describes and shows the distance as measured between the pair of current injecting electrodes 4. This distance (d) is according to the invention from 5mm to 12mm, preferably about 6 to 10 mm and more preferably about 7 to 9mm. The distance (d) should be more than 4 mm for reasons explained in relation to figure 3.

Still referring to figure 1b, the centers of the current injection electrodes 4 and the voltage sensing electrodes 5 are arranged flush with the convex surface and the voltage sensing electrodes are arranged symmetrically between the current injection electrodes 4. The distance (e) between a first injection electrode to sensing electrode is the same as the distance (e) between the second voltage sensing electrode to second current injection electrode. This distance (e) is optimally chosen to be around 1mm to 4mm, preferably about 2mm to 3mm and actually depends on the distance (d). The distance e may be calculated using the following formula:

$$e < d/2;$$

whereby e $\geq$ 1mm. thus (e) may preferably be smaller than half the distance (d) between the two injection electrodes.

The distances (d) and (e) may be measured on the surface of the housing 2 or they may be simplified and measured along a fictional straight line between the current injection electrodes.

The above-described distancing between the current injection electrodes 4 and the voltage sensing electrodes 5 works for any sensor shape and housing design. It is not limited to harmonic and rounded of shaped housings.

Figure 2a illustrates a similar embodiment of an implantable sensor 1' as figure 1 but in this case with circular current injecting electrodes 4'. All other features are the same or similar to the embodiment shown in figures 1a and 1b. The circular current injecting electrodes 4' work in a similar manner as the longitudinal current injection electrodes 4 shown with reference to figures 1a and 1b.

In the embodiments shown in figures 1a, 1b, 2a and 2b, the current injecting and voltage sensing electrodes 4, 4', 5 are arranged in a row on the outer surface of the first part 3a with the two voltage sensing electrodes 5 being arranged in between the two current injecting electrodes 4, 4'.

Figure 2a further illustrates the longitudinal axis (a) of the housing 2. In addition, figure 2a also illustrates the first half 11a and the second half 11b of the housing. The first half 11a and the second half 11b being defined by splitting the housing 2 in the middle as seen along the longitudinal axis (a) or split by a plane that extends perpendicular to the longitudinal axis (a) and which plane extends through a center of the housing 2.

Figure 2b also illustrates the distances (d') between the pair of injection electrodes **4'** and the distance (e') between the first current injection electrode and the first voltage sensing electrode and the distance (e'), which is the same, between the second voltage sensing electrode and the second voltage sensing electrode.

The reason for the above distancing details becomes clear when viewing figure 3. Figure 3 is a schematic representation that illustrates an electronic and geometric relationship between current trajectories and equipotential lines, in a cross-sectional view onto a plane that is cut through the electrodes 4, 4', 5 and the longitudinal axis (a). Figure 3 is to be understood to show illustratively the principle of a four-point measurement using a pair of current injecting electrodes 4" and a pair of voltage sensing electrodes 5'. The shape of the different curves illustrated in figure 3 is not limiting nor is it directly binding, it is used for illustration. Further, the surface along which the electrodes 4", 5' are placed, is shown to be flat and not curved as in figures 1a and 1b, for illustrative purposes. The principles described in relation to figure 3 also apply to curved surfaces, for example ellipsoidal surfaces. Figure 3 illustrates two current injecting electrodes 4" that extend electric field lines or current trajectories 14 from one current injecting electrode 4" to the other current injecting electrode 4" through organic tissue 16. The pair of current injecting electrodes 4" are connected to a current source (not shown). Figure 3 further illustrates a pair of voltage sensing electrodes 5' arranged symmetrically in between the pair of current injecting electrodes 4". The voltage sensing electrodes are designed to detect and measure impedance (voltage) between the equipotential lines 18 that define a volume (spheric surface) within the same voltage. The two voltage sensing electrodes 5' are connected to a voltmeter and therewith the impedance in the tissue can be determined and measured.

Figure 3 further illustrates the (fictional and simplified) line of the scar tissue (s) that is present when the sensor 1, 1' is implanted in organic tissue of a living being. One can now understand why the distance (d), (d') between the pair of current injecting electrodes 4" is of importance. If this distance (d), (d') is chosen to be too short then the current lines 14 will not extend into healthy tissue 16 and remain in the scar tissue 20 and therewith the voltage that is sensed between the equipotential lines 18 is only coming from the scar tissue 20 and the values will not be representative. If the distance (d), (d') between the pair of current injecting electrodes 4" is however chosen to be more than two times the thickness of the scar tissue 20 then the current trajectories 14 will start to extend into the healthy tissue 16 and the voltage sensing electrodes 5 can detect the voltage between equipotential lines that extend into the healthy tissue 16, as illustrated in figure 3. In figure 3, the distance (d), (d') between the pair of injection electrodes 4" is chosen to be about four times the thickness of the scar tissue 20, as measured perpendicular to the surface in which the electrodes 4", 5 are arranged, thus about 8mm.

The distance (d), (d') can however not be chosen to be as great as possible either, since a great distance between the current injecting electrodes will raise other issues such as measurements in inhomogeneous tissue and artefacts in the measurement due to movement of the living being during measurement. That is the reason why the above given ranges are suitable.

Likewise figure also illustrates the distance (e), (e') between the first current injection electrode 4" and the closest voltage sensing electrode 5. In the example of figure 3, this distance is about the thickness of the scar tissue 20, thus around 2mm.

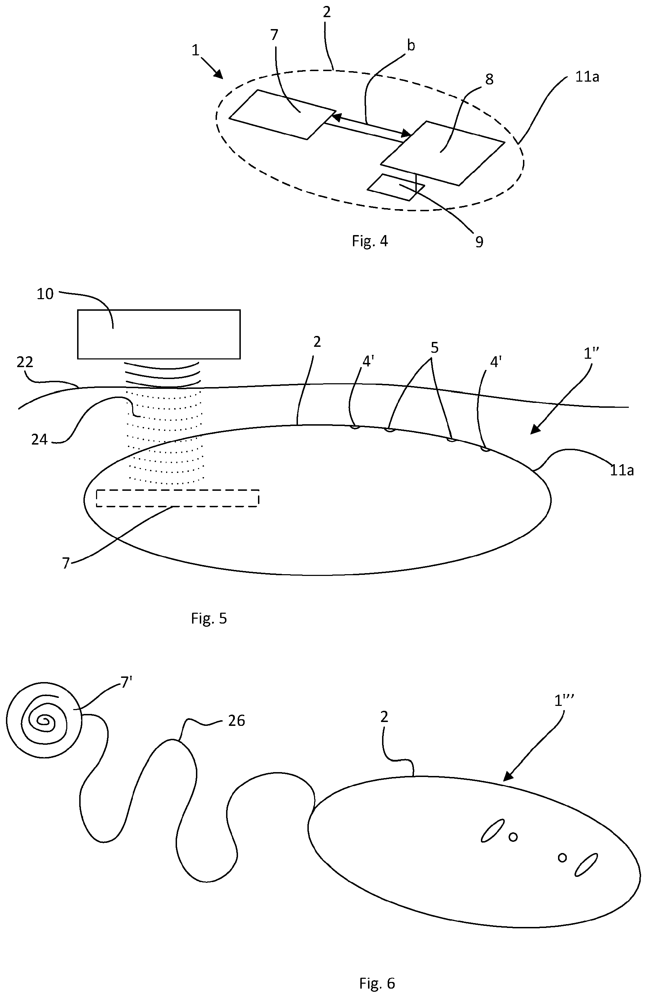

Figure 4 illustrates the interior of the implantable sensor 1, comprising a coil 7, a circuit board 8 and a communication unit 9, which are electrically connected to one another. The housing 2 is shown in dashed lines to illustrate that it is omitted for illustrative purposes and so are the electrodes. The coil 7 is arranged next to the circuit board 8 but not overlapping. The two current injecting electrodes 4, 4' and the two voltage sensing electrodes 5 are arranged on the first part 11a of the housing 2 that is arranged around the circuit board (not shown in figure 4 but c.f. figure 5). The two current injecting electrodes 4, 4' and the two voltage sensing electrodes 5 are thus arranged above the circuit board 8 and not above the coil 7. The two current injecting electrodes 4, 4' and the two voltage sensing electrodes 5 are further connected to the circuit board 8.

The positioning of the coil 7 away from the circuit board 8 by the distance (b) reduces the presence of Eddy Currents when the implantable glucose sensor 1, 1', 1", 1''' is powered by an external power source 10 as illustrated in figure 5.

Figure 5 illustrates the measuring of the impedance of the tissue surrounding the sensor 1" during powering of the implanted sensor 1" via an external coil 10. Varying electromagnetic waves 24 originating from the external device induce a current in the coil 7, which current then powers the sensor 1" for measuring impedance in the tissue/body fluid via the current injecting electrodes 4' and the voltage sensing electrodes 5. The coil 7 is shown in dashed lines as figure 5 is actually a cross sectional view onto a cross section cut through the tissue of a patient showing the implanted sensor 1" from the side. The external device 10 is arranged outside the body and skin 22 of the patient during powering of the sensor 1". Although not shown in figure 5, the external device may be positioned adjacent the skin 22 during measurement.

Alternatively, to figure 5, the sensor 1''' may comprise a coil 7' that is arranged outside the housing 2 of the sensor 1''' and connected to the electronics, such as the circuit board 8 of the sensor 1''' via a cable as shown in figure 6. The reason for using a coil 7' that is arranged separately from the housing 2 is that the coil can be placed close to the skin of a patient or living being so that it is easy for the external device 10 to power the sensor 1''' via the coil 7' (c.f. figure 5).

The coil 7' that is arranged outside the housing 2 may comprise means (not shown) for fixating it onto tissue within the body so that it cannot move or change its orientation after implantation.

The conductor 26 that is connecting the coil 7' with the electronics of the sensor 1''' may be a flexible cable coated with biocompatible material.

The embodiment shown in figure 6 can be embodied with the distancing of the current injecting and voltage sensing electrodes as described above, or it may be embodied without such a spacing of the electrodes and with another shaped housing 2. The idea or disclosure of having the coil 7' arranged outside the housing 2 can be embodied with other sensors as well and is not limited to the disclosure herein and to implantable sensors designed to measure glucose.

The invention has mainly been described above with reference to a few embodiments. However, as is readily appreciated by a person skilled in the art, other embodiment than the ones disclosed above are equally possible within the scope of the invention as defined by the appended patent claims.

**Claims**

1. An implantable glucose sensor (1, 1', 1", 1‴) for determining impedance in tissue of a living being, the implantable sensor comprising:

   - a housing (2, 2'),
   - two current injecting electrodes (4, 4') for injecting current into the tissue,
   - two voltage sensing electrodes (5) for measuring impedance in the tissue, whereby the two voltage sensing electrodes (5) are arranged separately from the two current injecting electrodes (4, 4'),
   - a coil (7) for powering the implantable glucose sensor via a power source (10),
   - a circuit board (8) arranged within the housing (2, 2'), the circuit board (8) being electrically connected to the two voltage sensing electrodes (5) and the two current injecting electrodes (4, 4') and the coil (7),
   - a communication unit (9) for transferring and receiving data packages, the communication unit (9) being connected to the circuit board (8),
   - the housing (2) comprising the two voltage sensing electrodes (5) and the two current injecting electrodes (4, 4') on an outer surface,

   **characterized in that** a distance (d, d') between the two current injecting electrodes (4, 4') on the outer surface is in a range from 5mm to 12mm, preferably about 6 to 10mm and more preferably about 7 to 9mm.

2. The implantable glucose sensor according to claim 1, wherein the relationship between the distance (d) between the two current injecting electrodes (4, 4') and a distance (e, e') between one of the two voltage sensing electrodes (5) and a closest injecting electrode (4) and a distance (e, e') between the other of the two voltage sensing electrodes and the other of the two current injecting electrodes (4'), respectively follows the equation:

$$e < d/2 \text{ and } e \geq 1mm.$$

3. The implantable glucose sensor according to claim 1 or 2, wherein the voltage sensing electrodes (5) are arranged in between the current injecting electrodes (4, 4') and wherein a distance (e, e') between one of the two voltage sensing electrodes (5) and a closest current injecting electrode (4) and a distance between the other of the two voltage sensing electrodes and a closest current injecting electrode (4') is in a range from 1mm to 4mm, preferably about 1.5mm to 3mm.

4. The implantable glucose sensor according to any of the previous claims, wherein the two current injecting electrodes (4, 4') comprise a first active area made of conductive material and wherein the voltage sensing electrodes (5) comprise a second active area made of conductive material, wherein the first active area is bigger than the second active area.

5. The implantable glucose sensor according to any of the previous claims, wherein the first active area and the second active area are made of a bio-compatible conductive material such as gold, titan, platinum or any alloy thereof.

6. The implantable glucose sensor according to any of the previous claims, wherein the two current injecting electrodes (4) have a longitudinal shape, such as for example an elliptic longitudinal shape or a rectangular longitudinal shape.

7. The implantable glucose sensor according to claim 1 or 2, wherein the housing (2 2') is shaped harmonic and rounded off and convex on a bottom side and on a top side.

8. The implantable glucose sensor according to any of the preceding claims, wherein the housing (2) is of an ellipsoid shape.

9. The implantable glucose sensor according to any of the previous claims, wherein outer dimensions of the implantable sensor correspond to a length of 10mm to 60mm, preferably 20mm to 40mm, a width of 5mm to 25mm, preferably 11mm to 15mm and a thickness of 1mm to 15mm, preferably 2mm to 5mm.

10. The implantable glucose sensor according to any of the previous claims, wherein the implantable sensor further comprises a flexible cable (26) and wherein the coil (7') is arranged outside the housing (2, 2') connected to the housing (2, 2') and the circuit board (8) via the flexible cable (26).

11. The implantable glucose sensor according to any of the previous claims, wherein the two current injecting electrodes and the two voltage sensing electrodes are arranged along a straight line, wherein the straight line is straight in two dimensions, such as in a top down view onto the implantable sensor.

12. The implantable glucose sensor according to any of the previous claims 1 to 10, wherein the two current injecting electrodes and the two voltage sensing electrodes are located on neighbouring corners respectively of a square or rectangle.

13. The implantable glucose sensor according to any of the previous claims 1 to 12, wherein the voltage sensing electrodes (5) are arranged synmmetrically in between the injecting electrodes (4, 4') and wherein a distance (e, e') between one of the two voltage sensing electrodes (5) and a closest current injecting electrode (4) and a distance between the other of the two voltage sensing electrodes and a closest current injecting electrode (4') is in a range from 1mm to 4mm, preferably about 1.5mm to 3mm.

14. The implantable glucose sensor according to any of the previous claims 1 to 13, wherein the implantable sensor is an implantable glucose sensor and wherein a glucose concertation in the tissue is determined by correlating a measured impedance to a database containing impedance values and corresponding glucose concentrations.

**Patentansprüche**

1. Implantierbarer Glukosesensor (1, 1', 1", 1''') zum Bestimmen von Impedanz in Gewebe eines Lebewesens, wobei der implantierbare Sensor Folgendes umfasst:

> - ein Gehäuse (2, 2'),
> - zwei Stromeinspeiseelektroden (4, 4') zum Einspeisen von Strom in das Gewebe,
> - zwei Spannungserfassungselektroden (5) zum Messen von Impedanz in dem Gewebe, wobei die zwei Spannungserfassungselektroden (5) getrennt von den zwei Stromeinspeiseelektroden (4, 4') angeordnet sind,
> - eine Spule (7) zum Speisen des implantierbaren Glukosesensors über eine Stromquelle (10),
> - eine innerhalb des Gehäuses (2, 2') angeordnete Leiterplatte (8), wobei die Leiterplatte (8) mit den zwei Spannungserfassungselektroden (5) und den zwei Stromeinspeiseelektroden (4, 4') und der Spule (7) elektrisch verbunden ist,
> - eine Kommunikationseinheit (9) zum Übertragen und Empfangen von Datenpaketen, wobei die Kommunikationseinheit (9) mit der Leiterplatte (8) verbunden ist,
> - das Gehäuse (2) die zwei Spannungserfassungselektroden (5) und die zwei Stromeinspeiseelektroden (4, 4') auf einer Außenfläche umfasst,
>
> **dadurch gekennzeichnet, dass** ein Abstand (d, d') zwischen den zwei Stromeinspeiseelektroden (4, 4') auf der Außenfläche in einem Bereich von 5 mm bis 12 mm, vorzugsweise etwa 6 bis 10 mm und noch bevorzugter etwa 7 bis 9 mm liegt.

2. Implantierbarer Glukosesensor nach Anspruch 1, wobei die Beziehung zwischen dem Abstand (d) zwischen den zwei Stromeinspeiseelektroden (4, 4') und einem Abstand (e, e') zwischen einer der zwei Spannungserfassungselektroden (5) und einer nächstgelegenen Einspeiseelektrode (4) bzw. einem Abstand (e, e') zwischen der anderen der zwei Spannungserfassungselektroden und der anderen der zwei Stromeinspeiseelektroden (4') jeweils der folgenden Gleichung folgt:

$$e < d/2 \text{ und } e \geq 1mm.$$

3. Implantierbarer Glukosesensor nach Anspruch 1 oder 2, wobei die Spannungserfassungselektroden (5) zwischen den Stromeinspeiseelektroden (4, 4') angeordnet sind und wobei ein Abstand (e, e') zwischen einer der zwei Spannungserfassungselektroden (5) und einer nächstgelegenen Stromeinspeiseelektrode (4) sowie ein Abstand zwischen der anderen der zwei Spannungserfassungselektroden und einer nächstgelegenen Stromeinspeiseelektrode (4') in einem Bereich von 1 mm bis 4 mm, vorzugsweise etwa 1,5 mm bis 3 mm liegt.

4. Implantierbarer Glukosesensor nach einem der vorhergehenden Ansprüche, wobei die zwei Stromeinspeiseelektroden (4, 4') eine aus leitfähigem Material hergestellte erste aktive Fläche umfassen und wobei die Spannungserfassungselektroden (5) eine aus leitfähigem Material hergestellte zweite aktive Fläche umfassen, wobei die erste aktive Fläche größer ist als die zweite aktive Fläche.

5. Implantierbarer Glukosesensor nach einem der vorhergehenden Ansprüche, wobei die erste aktive Fläche und die zweite aktive Fläche aus einem biokompatiblen leitfähigen Material, wie Gold, Titan, Platin oder einer beliebigen Legierung davon, hergestellt sind.

6. Implantierbarer Glukosesensor nach einem der vorhergehenden Ansprüche, wobei die zwei Stromeinspeiseelektroden (4) eine längliche Form aufweisen, wie beispielsweise eine elliptische längliche Form oder eine rechteckige längliche Form.

7. Implantierbarer Glukosesensor nach Anspruch 1 oder 2, wobei das Gehäuse (2, 2') harmonisch geformt und an einer Unterseite und an einer Oberseite abgerundet und konvex ist.

8. Implantierbarer Glukosesensor nach einem der vorhergehenden Ansprüche, wobei das Gehäuse (2) eine ellipsoide Form aufweist.

9. Implantierbarer Glukosesensor nach einem der vorhergehenden Ansprüche, wobei Außenabmessungen des implantierbaren Sensors einer Länge von 10 mm bis 60 mm, vorzugsweise 20 mm bis 40 mm, einer Breite von 5 mm bis 25 mm, vorzugsweise 11 mm bis 15 mm und einer Dicke von 1 mm bis 15 mm, vorzugsweise 2 mm bis 5 mm, entsprechen.

10. Implantierbarer Glukosesensor nach einem der vorhergehenden Ansprüche, wobei der implantierbare Sensor ferner ein flexibles Kabel (26) umfasst und wobei die Spule (7') außerhalb des Gehäuses (2, 2'), über das flexible Kabel (26) mit dem Gehäuse (2, 2') und der Leiterplatte (8) verbunden, angeordnet ist.

11. Implantierbarer Glukosesensor nach einem der vorhergehenden Ansprüche, wobei die zwei Stromeinspeiseelektroden und die zwei Spannungserfassungselektroden entlang einer geraden Linie angeordnet sind, wobei die gerade Linie in zwei Dimensionen, wie in einer Draufsicht auf den implantierbaren Sensor, gerade ist.

12. Implantierbarer Glukosesensor nach einem der vorhergehenden Ansprüche 1 bis 10, wobei sich die zwei Stromeinspeiseelektroden und die zwei Spannungserfassungselektroden jeweils an benachbarten Ecken eines Quadrats oder Rechtecks befinden.

13. Implantierbarer Glukosesensor nach einem der vorhergehenden Ansprüche 1 bis 12, wobei die Spannungserfassungselektroden (5) symmetrisch zwischen den Einspeiseelektroden (4, 4') angeordnet sind und wobei ein Abstand (e, e') zwischen einer der zwei Spannungserfassungselektroden (5) und einer nächstgelegenen Stromeinspeiseelektrode (4) sowie ein Abstand zwischen der anderen der zwei Spannungserfassungselektroden und einer nächstgelegenen Stromeinspeiseelektrode (4') in einem Bereich von 1 mm bis 4 mm, vorzugsweise etwa 1,5 mm bis 3 mm liegt.

14. Implantierbarer Glukosesensor nach einem der vorhergehenden Ansprüche 1 bis 13, wobei es sich bei dem implantierbaren Sensor um einen implantierbaren Glukosesensor handelt und wobei eine Glukosekonzentration in dem Gewebe durch Korrelieren einer gemessenen Impedanz mit einer Datenbank, die Impedanzwerte und entsprechende Glukosekonzentrationen enthält, bestimmt wird.

**Revendications**

1. Capteur de glucose implantable (1, 1', 1", 1‴) pour déterminer une impédance dans un tissu d'un être vivant, le capteur de glucose implantable comprenant :

   - un boîtier (2, 2'),
   - deux électrodes d'injection de courant (4, 4') pour injecter du courant dans le tissu,
   - deux électrodes de détection de tension (5) pour mesurer une impédance dans le tissu, moyennant quoi les deux électrodes de détection de tension (5) sont agencées séparément des deux électrodes d'injection de courant (4, 4'),
   - une bobine (7) pour alimenter le capteur de glucose implantable via une source d'alimentation (10),
   - une carte de circuit imprimé (8) agencée à l'intérieur du boîtier (2, 2'), la carte de circuit imprimé (8) étant connectée électriquement aux deux électrodes de détection de tension (5) et aux deux électrodes d'injection de courant (4, 4') et à la bobine (7),
   - une unité de communication (9) pour transférer et recevoir des paquets de données, l'unité de communication (9) étant connectée à la carte de circuit imprimé (8),
   - le boîtier (2) comprenant les deux électrodes de détection de tension (5) et les deux électrodes d'injection de courant (4, 4') sur une surface externe,

   **caractérisé en ce qu'une** distance (d, d') entre les deux électrodes d'injection de courant (4, 4') sur la surface externe est dans une plage allant de 5 mm à 12 mm, de préférence d'environ 6 à 10 mm et de manière davantage préférée d'environ 7 à 9 mm.

2. Capteur de glucose implantable selon la revendication 1, dans lequel la relation entre la distance (d) entre les deux électrodes d'injection de courant (4, 4') et une distance (e, e') entre l'une des deux électrodes de détection de tension (5) et une électrode d'injection la plus proche (4) et une distance (e, e') entre l'autre des deux électrodes de détection de tension et l'autre des deux électrodes d'injection de courant (4'), respectivement, suit l'équation :

$$e < d/2 \text{ et } e \geq 1 \text{ mm.}$$

3. Capteur de glucose implantable selon la revendication 1 ou 2, dans lequel les électrodes de détection de tension (5) sont agencées entre les électrodes d'injection de courant (4, 4') et dans lequel une distance (e, e') entre l'une des deux électrodes de détection de tension (5) et une électrode d'injection de courant la plus proche (4) et une distance entre l'autre des deux électrodes de détection de tension et une électrode d'injection de courant la plus proche (4') est dans une plage allant de 1 mm à 4 mm, de préférence d'environ 1,5 mm à 3 mm.

4. Capteur de glucose implantable selon l'une quelconque des revendications précédentes, dans lequel les deux électrodes d'injection de courant (4, 4') comprennent une première zone active constituée d'un matériau conducteur et dans lequel les électrodes de détection de tension (5) comprennent une seconde zone active constituée d'un matériau conducteur, dans lequel la première zone active est plus grande que la seconde zone active.

5. Capteur de glucose implantable selon l'une quelconque des revendications précédentes, dans lequel la première zone active et la seconde zone active sont constituées d'un matériau conducteur biocompatible tel que l'or, le titane, le platine ou tout alliage de ceux-ci.

6. Capteur de glucose implantable selon l'une quelconque des revendications précédentes, dans lequel les deux électrodes d'injection de courant (4) ont une forme longitudinale, comme par exemple une forme longitudinale elliptique ou une forme longitudinale rectangulaire.

7. Capteur de glucose implantable selon la revendication 1 ou 2, dans lequel le boîtier (2, 2') est de forme harmonique, et est arrondi et convexe sur un côté inférieur et sur un côté supérieur.

8. Capteur de glucose implantable selon l'une quelconque des revendications précédentes, dans lequel le boîtier (2) est de forme ellipsoïdale.

9. Capteur de glucose implantable selon l'une quelconque des revendications précédentes, dans lequel les dimensions externes du capteur de glucose implantable correspondent à une longueur de 10 mm à 60 mm, de préférence de 20 mm à 40 mm, une largeur de 5 mm à 25 mm, de préférence de 11 mm à 15 mm et une épaisseur de 1 mm à 15 mm, de préférence de 2 mm à 5 mm.

10. Capteur de glucose implantable selon l'une quelconque des revendications précédentes, dans lequel le capteur implantable comprend également un câble flexible (26) et dans lequel la bobine (7') est agencée à l'extérieur du boîtier (2, 2'), connectée au boîtier (2, 2') et à la carte de circuit imprimé (8) via le câble flexible (26).

11. Capteur de glucose implantable selon l'une quelconque des revendications précédentes, dans lequel les deux électrodes d'injection de courant et les deux électrodes de détection de tension sont agencées le long d'une ligne droite, dans lequel la ligne droite est droite dans deux dimensions, comme dans une vue de dessus du capteur implantable.

12. Capteur de glucose implantable selon l'une quelconque des revendications précédentes 1 à 10, dans lequel les deux électrodes d'injection de courant et les deux électrodes de détection de tension sont situées respectivement sur des coins voisins d'un carré ou d'un rectangle.

13. Capteur de glucose implantable selon l'une quelconque des revendications précédentes 1 à 12, dans lequel les électrodes de détection de tension (5) sont agencées symétriquement entre les électrodes d'injection (4, 4') et dans lequel une distance (e, e') entre l'une des deux électrodes de détection de tension (5) et une électrode d'injection de courant la plus proche (4) et une distance entre l'autre des deux électrodes de détection de tension et une électrode d'injection de courant la plus proche (4') est dans une plage allant de 1 mm à 4 mm, de préférence d'environ 1,5 mm à 3 mm.

14. Capteur de glucose implantable selon l'une quelconque des revendications précédentes 1 à 13, dans lequel le capteur implantable est un capteur de glucose implantable et dans lequel une concentration de glucose dans le tissu est déterminée en corrélant une impédance mesurée à une base de données contenant des valeurs d'impédance et des concentrations de glucose correspondantes.

Fig. 1a

Fig. 1b

Fig. 2a

Fig. 2b

Fig. 3

Fig. 4

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5970986 B1

- US 2015196224 A1